(19) **Europäisches Patentamt**

**European Patent Office**

**Office européen des brevets**

(11) Veröffentlichungsnummer: **0 048 347**
**B1**

(12) **EUROPÄISCHE PATENTSCHRIFT**

(45) Veröffentlichungstag der Patentschrift:
27.07.83

(51) Int. Cl.³: **C 12 Q 1/00,** C 12 Q 1/26,
G 01 N 33/52

(21) Anmeldenummer: 81106565.5

(22) Anmeldetag: 24.08.81

(54) Verfahren und Reagens zur Bestimmung von Glycerin.

(30) Priorität: 19.09.80 DE 3035465

(43) Veröffentlichungstag der Anmeldung:
31.03.82 Patentblatt 82/13

(45) Bekanntmachung des Hinweises auf die Patenterteilung:
27.07.83 Patentblatt 83/30

(84) Benannte Vertragsstaaten:
AT BE CH DE FR GB IT LI LU NL SE

(56) Entgegenhaltungen:
EP-A-0 008 341
EP-A-0 010 296
EP-A-0 033 540
DE-A-2 130 340
DE-A-2 817 087
US-A-4 038 146
US-A-4 220 503
US-A-4 241 178
RESEARCH DISCHLOSURE, Nr. 161, Nr. 16 146,
September 1977, Seiten 94—98 »Integral element
for the detection of glycerol or triglycerides«

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Ziegenhorn, Joachim, Dr., Ina-Seidel-Weg 1,
D-8130 Starnberg (DE)
Erfinder: Bartl, Knut, Dr., Am Westend 6,
D-8121 Wilzhofen (DE)
Erfinder: Röder, Albert, Dr., Bahnhofstrasse 41,
D-8124 Seeshaupt (DE)
Erfinder: Lang, Gunter, Lange Strasse 22,
D-8132 Tutzing (DE)
Erfinder: Möllering, Hans, Herrestrasse 10,
D-8132 Tutzing (DE)
Erfinder: Nägele, Ulrich, Dr., Bahnhofstrasse 6,
D-8131 Bernried (DE)

(74) Vertreter: Weickmann, Heinrich, Dipl.-Ing. et al,
Patentanwälte Dipl.-Ing. H. Weickmann Dipl.-Phys.Dr. K.
Fincke Dipl.-Ing. F.A. Weickmann Dipl.-Chem. B. Huber
Dr.-Ing. H. Liska Möhlstrasse 22,
D-8000 München 86 (DE)

CHEMICAL ABSTRACTS, Band 87, Nr. 21, 21.
November 1977, Seite 604, Nr. 168 288v Columbus,
Ohio, U.S.A. J. H. PAZUR et al.: »The oxidation of
aldoses, alditols and sugar nucleotides by galactose oxidase«

## Verfahren und Reagens zur Bestimmung von Glycerin

Die Erfindung betrifft ein Verfahren zur enzymatischen Bestimmung von Glycerin und ein Reagens zur Durchführung dieses Verfahrens.

Die Bestimmung von Triglyceriden (=Glycerinester von langkettigen Fettsäuren) hat für die medizinische Diagnostik eine erhebliche Bedeutung. Ein erhöhter Triglyceridspiegel im Blut ist ein wesentlicher Risikofaktor der Arteriosklerose. Bei hohen Triglyceridwerten, also Hypertriglyceridämie, kommen Koronarinsuffizienz und Herzinfarkt häufiger vor als bei niedrigen Triglyceridwerten. Hypertriglyceridämie begünstigt das Auftreten von Arteriosklerose und Koronarerkrankungen und muß daher frühzeitig erkannt werden, damit die Behandlung rechtzeitig beginnen kann. Eine rasche und zuverlässige durchführbare Methode zur Bestimmung von Triglyceriden bzw. Glycerin hat daher große Bedeutung.

Die bekannten und brauchbaren Methoden zur Triglyceridbestimmung basieren auf der enzymatischen Hydrolyse der Triglyceride mittels Lipase/Esterase und Bestimmung des freigesetzten Glycerins mittels Glycerokinase/Pyruvatkinase/Lactatdehydrogenase (A. W. Wahlefeld, in Methods of Enzymatic Analysis IV, H. U. Bergmeyer, Ed. Academic Press, New York, London, 1974, S. 1831 — 1835).

Dieses bekannte Verfahren weist jedoch wesentliche Nachteile auf. Um Störungen durch Eigenfärbungen des Serums (Bilirubin, Hämoglobin) sowie Trübungen zu eliminieren, müssen stets Probenleerwert-Bestimmungen durchgeführt werden, was den Zeitbedarf pro Analyse sowie den Reagensverbrauch und damit die Kosten erhöht. Ferner ergeben sich infolge der Anwesenheit zahlreicher empfindlicher Coenzyme und Enzyme nur relativ geringe Haltbarkeiten für die Gebrauchslösungen.

Neben diesem UV-Test sind auch Farbtests auf Basis einer Formazan-Bildung bekannt. Letztere erlauben zwar die Bestimmung gegen einen Reagenzien-Leerwert, sind jedoch relativ kompliziert aufgebaut und störanfällig.

Schließlich ist ein Verfahren unter Verwendung einer Glycerinoxidase bekannt, welche $H_2O_2$ und Glycerinaldehyd bildet, die bestimmt werden können. Dieses Enzym wird jedoch entweder aus hochpathogenen Mikroorganismen, wie Pestbazillen, oder nur in recht geringen Ausbeuten aus anderen Mikroorganismen erhalten.

Es besteht daher ein Bedarf an einem Verfahren und einem Reagens, welches die oben geschilderten Nachteile nicht aufweist. Insbesondere besteht ein Bedarf an einem Verfahren, welches mit einem einfach in guter Ausbeute zugänglichen Enzym durchgeführt werden kann und welches auch ohne Messung gegen Probenleerwerte ausreichend genaue Ergebnisse liefert. Insbesondere soll ein derartiges Verfahren als Indikatorreaktion eine Farbreaktion mit ausreichend großem Extinktionskoeffizienten des gebildeten Farbstoff ($\varepsilon > 18$ cm$^2$/μMol) verwenden können.

Gelöst wird diese Aufgabe erfindungsgemäß durch ein Verfahren zur Bestimmung von Glycerin, welches darin besteht, daß Glycerin in wäßrigem Medium mit Galactoseoxidase inkubiert und entweder der Sauerstoffverbrauch oder gebildetes $H_2O_2$ bzw. Glycerinaldehyd bestimmt werden.

Galactoseoxidase EC 1.1.3.9 ist ein bekanntes Enzym, welches in Gegenwart von Sauerstoff D-Galactose zu D-Galactosehexodialdose und $H_2O_2$ oxidiert. Es handelt sich um ein kupferhaltiges Enzym. Die Erfindung beruht auf der überraschenden Feststellung, daß sich dieses Enzym gut zur quantitativen Bestimmung von Glycerin verwenden läßt. Dies war nicht zu erwarten, da zwar eine minimale Aktivität der Galactoseoxidase (im folgenden als Gal-OD bezeichnet) gegenüber Glycerin bekannt war, aber nach Hamilton et al. »Oxidases and Related Redox Systems«, Ed. King, Bd. 1 (1971) die Umsatzrate nur ein 150stel, bezogen auf Galactose beträgt. Da in Körperflüssigkeiten, in denen die Glycerinbestimmung erwünscht ist, auch Galactose in freier oder gebundener Form vorkommen kann, war eine unerträglich starke Störung durch Galactose zu erwarten gewesen, ganz abgesehen davon, daß nach den bekannten Befunden eine quantitative Umsetzung des Glycerins nicht vorhersehbar war. Überraschenderweise wurde jedoch gefunden, daß in Körperflüssigkeiten, insbesondere in Serum, die befürchtete Störung nicht auftritt.

Das Verfahren der Erfindung kann auch bei vorheriger oder gleichzeitiger enzymatischer Verseifung von Triglyceriden unter Freisetzung von Glycerin oder bei vorheriger chemischer Verseifung von Triglyceriden durchgeführt werden. Zur chemischen Verseifung eignet sich beispielsweise alkoholische Kalilauge, zur enzymatischen Verseifung Lipase mit Esterase oder Esterase allein.

Die Messung des Sauerstoffverbrauchs, der $H_2O_2$-Bildung oder der Glycerinaldehyd-Bildung kann nach den hierfür bekannten Methoden erfolgen.

Geeignete Methoden zur Bestimmung des Sauerstoffverbrauchs sind beispielsweise Gaschromatographie und Depolarisationsverfahren. Bevorzugt wird die polarometrische Bestimmung mittels Sauerstoffelektrode, da sich dieses Verfahren besonders zur automatischen Durchführung der Bestimmung eignet. Derartige Bestimmungsmethoden sind bekannt. Besonders geeignet erwiesen sich die in den deutschen Offenlegungsschriften 2 130 340 und 2 130 308 beschriebenen Methoden zur polarometrischen Messung des Sauerstoffverbrauchs in wäßrigen Medien. Gebildetes Wasserstoffperoxid kann sowohl titrimetrisch als auch potentiometrisch, polarographisch und colorimetrisch sowie enzymatisch bestimmt werden. Bevorzugt werden die enzymatischen Methoden unter Verwendung von Katalase oder Peroxidase, da diese nicht nur äußerst spezifisch und zuverlässig sind, sondern auch mit der Hauptreaktion unter Bildung von Wasserstoffperoxid auf einfachste Weise kombiniert werden können. Als geeignet erwies sich auch die Bestimmung mittels Katalase in Gegenwart von $\beta$-Diketonen, z. B. Acetylaceton und Methanol bzw. Äthanol oder Methylenglykol, sowie die Bestimmung mittels Peroxidase in Gegenwart eines oder mehrerer Chromogene. Bei der Bestimmung mittels Katalase, Acetylaceton und Methanol wird letzteres zu Formaldehyd oxidiert, der mit Acetylaceton eine Farbreaktion eingeht, die gemessen werden kann. Bei der Bestimmung mittels Peroxidase werden als Chromophor Verbindungen eingesetzt, die nach der Reaktion photometrisch bestimmt werden können. Ein Beispiel für ein geeignetes Chromophor ist 2,2'-Azinobenzthiazolinsulfonsäure (ABTS). Ein anderes Beispiel ist das Indikatorsystem nach Trinder (Ann. Clin. Biochem. 6 (1969), 24—27), bei dem Phenol und 4-Aminoantipyrin (4-AAP) in Gegenwart von POD und unter der Einwirkung von $H_2O_2$ oxidativ zu einem Farbstoff gekuppelt werden. Anstelle von Phenol können andere aromatische Alkohole, wie p-Chlorphenol, Anilinderivate, Naphthol, Naphtholderivate, Naphthylamin, Naphthylaminderivate, Aminochinoline, Hydroxychinoline, Dihydroxyphenylessigsäure und ähnlich reagierende Substanzen eingesetzt werden. Anstelle von 4-Aminoantipyrin können 4-Aminoantipyrin-Derivate, wie 4-Aminoantipyrinamid, Phenylendiaminsulfonsäure, MBTH (Methylbenzothiazolonhydrazon) S-MBTH (sulfoniertes Methylbenzothiazolonhydrazon), MBTH- und S-MBTH-Derivate sowie ähnlich reagierende Verbindungen eingesetzt werden.

Die Bestimmung des Glycerinaldehyds erfolgt mit Hilfe von Aldehydreagenzien, vorzugsweise einem mit Aldehydgruppen unter Hydrazonbildung umsetzenden Hydrazinderivat, wie z. B. 2,4-Dinitrophenylhydrazin. Das gebildete Hydrazon kann dann colorimetrisch bestimmt werden.

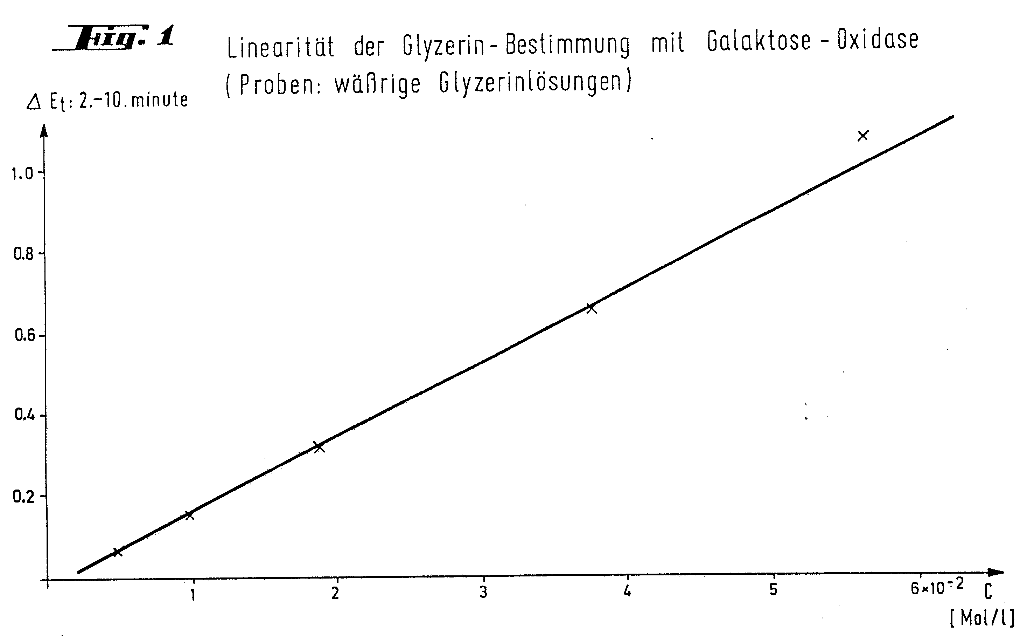

Als besonders geeignet und daher im Rahmen der Erfindung bevorzugt wird die Umsetzung in Gegenwart von Peroxidase und einem Chromogen, wie ABTS. Es wurde hier auch bei kurzen Reaktionszeiten ein linearer Zusammenhang zwischen Extinktionsdifferenz und Glycerinkonzentration festgestellt, so daß sich diese Ausführungsform des erfindungsgemäßen Verfahrens besonders zur kinetischen Bestimmung eignet, aber auch für Endpunktbestimmungen verwendbar ist. Die erzielte Proportionalität zwischen Extinktion und Glyceringehalt ist in der Zeichnung in Abbildung 1 dargestellt. Abbildung 1 zeigt in graphischer Darstellung den Zusammenhang zwischen Extinktion und Glyceringehalt.

Gemäß einer bevorzugten Ausführung der Erfindung erfolgt die Bestimmung in Gegenwart von Glycerindehydrogenase und ihrem Coenzym NAD. Glycerindehydrogenase EC 1.1.1.6 ist bekannt und beispielsweise aus Mikroorganismen, wie Enterobacter aerogenes erhältlich und handelsüblich. Es wurde gefunden, daß in Gegenwart von Glycerindehydrogenase (Glyc-DH) die Empfindlichkeit und Spezifität der Bestimmungsmethode noch weiter verbessert wird. Bei dieser bevorzugten Ausführungsform der Erfindung kann NAD im Überschuß über die stöchiometrisch benötigte Menge zugesetzt werden, vorzugsweise gibt man jedoch lediglich katalytische Mengen NAD und ein NAD-regenerierendes System zu. Geeignete NAD-regenerierende Systeme sind beispielsweise beschrieben in der DE-A-2 834 705. Ein bevorzugtes NAD-regenerierendes System zur Verwendung im Rahmen der Erfindung besteht aus Lactatdehydrogenase und ihrem Substrat Pyruvat. Wird NAD im Überschuß zugesetzt, so kann anstelle von $H_2O_2$ auch reduziertes NAD optisch gemessen werden.

Bei dieser Ausführungsform der Erfindung sollte der pH-Wert zwischen 7,0 und 9,0 eingestellt werden, da in diesem Bereich sämtliche beteiligten Enzyme, also Galactoseoxidase (Gal-OD), Glyc-DH und gegebenenfalls Peroxidase (POD) eine für das Verfahren gut geeignete Aktivität aufweisen.

Überraschenderweise verläuft die Umsetzung in dieser Ausführungsform glatt und quantitativ unter Verbrauch von molekularem Sauerstoff und Bildung von $H_2O_2$, obwohl die beiden Enzyme Gal-OD und Glyc-DH um das Glycerin konkurrieren und die Glyc-DH weder Sauerstoff verbraucht noch $H_2O_2$ bildet.

Durch die bevorzugte kombinierte Anwendung von Gal-OD und Glyc-DH wird der Empfindlichkeitsbereich mindestens bis 0,001 Mol/l herabgesetzt.

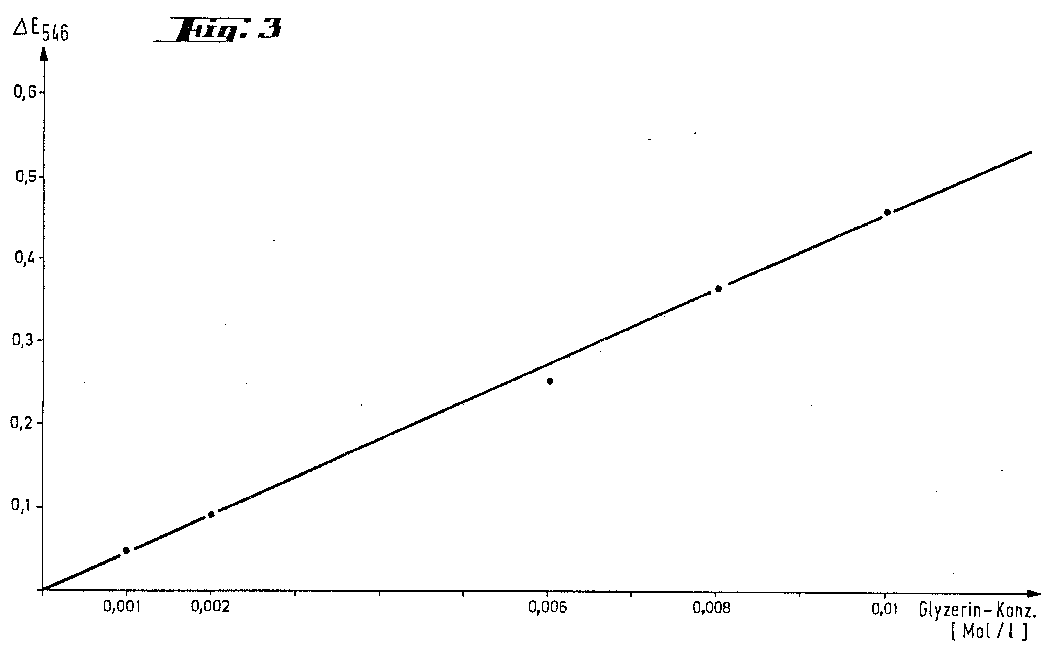

Die Durchführung der Messung kann sowohl nach dem Endwertverfahren gegen einen Reagenzienleerwert oder nach dem kinetischen Verfahren erfolgen. Abbildung 3 der Zeichnung zeigt, daß auch bei dieser Ausführungsform ein linearer Zusammenhang zwischen Glycerinkonzentration und Extinktionsdifferenz mindestens bis herab zu Glycerinkonzentrationen von 0,0005 Mol/l gegeben ist.

Auch diese Ausführungsform des erfindungsgemäßen Verfahrens kann unter gleichzeitiger enzymatischer Verseifung von Triglyceriden, bei welcher Glycerin freigesetzt und erfindungsgemäß sofort bestimmt wird, durchgeführt werden.

Das Verfahren eignet sich daher besonders auch für Analysenautomaten, da sich durch Eichung mit einem einzigen Standard die Glycerinkonzentration exakt bestimmen läßt.

Gegenstand der Erfindung ist weiter ein Reagens zur Bestimmung von Glycerin, welches aus Galactoseoxidase und einem System zur Bestimmung von $H_2O_2$ und einem System zur Bestimmung von $H_2O_2$ oder einem System zur Bestimmung von Glycerinaldehyd besteht. In einer ersten bevorzugten Ausführungsform besteht dieses Reagens im wesentlichen aus Galactoseoxidase, Peroxidase, wenigstens einem Chromogen und Puffer, einzeln oder gemischt. Als Chromogen wird ABTS bevorzugt. Ein anderes bevorzugtes Farbindikator-System ist das System nach Trinder.

In einer weiteren bevorzugten Ausführungsform besteht das Reagens im wesentlichen aus Galactoseoxidase, Katalase, Acetylaceton, Methanol und Puffer, einzeln oder gemischt.

In einer weiteren bevorzugten Ausführungsform besteht das erfindungsgemäße Reagens im wesentlichen aus Galactoseoxidase und einem mit Aldehydgruppen unter Hydrazonbildung reagierenden Hydrazinderivat sowie einem Puffer. Als Hydrazinderivat wird 2,4-Dinitrophenyl-hydrazin bevorzugt.

Die obenerwähnten bevorzugten Reagenskombinationen können außer den aufgeführten obligaten Bestandteilen zusätzlich übliche Lösungsmittel, Stabilisatoren oder/und oberflächenaktive Substanzen enthalten. Alle diese Zusatzstoffe sind dem Fachmann bekannt und in Nachweissystemen für Wasserstoffperoxid bzw. Glycerinaldehyd üblich. Vorzugsweise enthalten die erfindungsgemäßen Reagenzien zusätzlich noch lipolytische Enzyme, wie Lipase/Esterase oder Esterase allein, so daß sie auch zur Bestimmung von gebundenem Glycerin, welches in Form von Triglyceriden vorliegt, eingesetzt werden können.

Das Reagens enthält zweckmäßigerweise 0,5 bis 100 U/ml Peroxidase, 5 bis 35 U/ml Galactoseoxidase, 0,05 bis 0,2 mMol/ml Puffer, pH 6 bis 9, und 0,05 bis 20 μMol/ml Chromogen.

Als Chromogen kann das Reagens 5 bis 15 μMol/ml p-Chlorphenol und 0,1 bis 1,0 μMol/ml 4-Aminoantipyrinamid enthalten.

Das Reagens kann auf einem Trägermaterial, wie Papier, imprägniert vorliegen.

Vorzugsweise enthalten die obenerwähnten Reagenskombinationen die essentiellen Bestandteile in folgenden Mengenverhältnissen:

1) 15 bis 30 U/ml Galactoseoxidase
   0,5 bis 100 μ/ml Peroxidase
   0,05 bis 20 μMol/ml Chromogen
   sowie gegebenenfalls
   0,001 bis 0,1 g/ml oberflächenaktives Mittel und
   Puffer, pH 6 bis 9;

2) 15 bis 30 U/ml Galactoseoxidase
   1 bis 10 μMol/l 2,4-Dinitrophenylhydrazin
   und gegebenenfalls
   0,001 bis 0,1 g/ml oberflächenaktives Mittel und
   Puffer, pH 6 bis 9.

In einer bevorzugten Ausführungsform enthält das erfindungsgemäße Reagens zusätzlich Glyc-DH, NAD und gegebenenfalls ein NAD-regenerierendes System.

Für die NAD-regenerierenden Systeme gilt das oben zum Verfahren Mitgeteilte in gleicher Weise. Das bevorzugte NAD-regenerierende System besteht aus Lactatdehydrogenase (LDH) und Pyruvat. Die Gegenwart des NAD-regenerierenden Systems macht es möglich, mit einer katalytischen Menge des Coenzyms NAD für die Glyc-DH auszukommen, da im Laufe der Reaktion gebildetes NADH sofort durch das regenerierende System wieder zu NAD aufoxidiert wird. In Gegenwart eines derartigen NAD-regenerierenden Systems kann daher die NAD-Menge bis herab zu 0,05 μMol/ml betragen.

In dieser bevorzugten Ausführungsform des erfindungsgemäßen Reagens beträgt die Glyc-DH-Menge zweckmäßig 5 bis 100 U/ml. Natürlich können auch größere Mengen zugesetzt werden, dies ist jedoch weniger wirtschaftlich. Mengen unter 5 U/ml sind verwendbar, die durch den Glyc-DH erzielten Vorteile treten dann aber nicht mehr in vollem Umfang auf.

Geeignete Puffer lassen sich durch einfache Vorversuche feststellen. Als gut geeignet haben sich Kaliumphosphat-, Glycylglycin- und Bicin-Puffer (N,N-Bis(2-hydroxyäthyl)-glycin) in Konzentrationen zwischen 0,05 und 0,2 Mol/l erwiesen. Die oben angegebenen Enzymeinheiten für Galactoseoxidase beziehen sich auf Galactose als Substrat, wie beispielsweise von Pazur et al. in »Carbohydrates, Nucleosides, Nucleotides« 4, 147 (1977) beschrieben.

Die folgenden Beispiele erläutern die Erfindung weiter.

Beispiel 1

Proben: Wäßrige Glycerinlösungen.
Reagens: Kaliumphosphat-Puffer (0,1 Mol/l, pH 7,5) Peroxidase (12 500 U/l), Galactoseoxidase (20 000 U/l), ABTS (2 mMol/l).
Probe: Wäßrige Glycerinlösung im Bereich von 0,005 bis 0,06 Mol/l.
Bestimmungsansatz:
Meßstrahlung: Hg 405 nm; Schichtdicke der Küvette: 1 cm;
Inkubationstemperatur: 25°C.

2,0 ml Reagens in Küvette pipettieren, 0,1 ml Probe zusetzen und mischen. Extinktionsänderung von der zweiten bis zur zehnten Minute registrieren ($\Delta E_{2.-10.}$). Für die quantitative Glycerinbestimmung

4

0 048 347

wird dieses $\Delta$ E über einen Standard zur Glycerinkonzentration in Beziehung gesetzt.

## Beispiel 2

### Bestimmung von Glycerin in Serum

Reagens: Kaliumphosphat-Puffer (0,1 Mol/l, pH 7,5) Peroxidase (12 500 U/l), Galactoseoxidase 20 000 U/l), ABTS (2 mMol/l).

Probe: Als Probe wird Serum eingesetzt, dem unterschiedliche Mengen Glycerin im Bereich von 0,006 bis 0,07 Mol/l zugeführt wurden.

Bestimmungsansatz:
Meßstrahlung: Hg 405 nm; Schichtdicke der Küvette: 1 cm;
Inkubationstemperatur: 25° C.

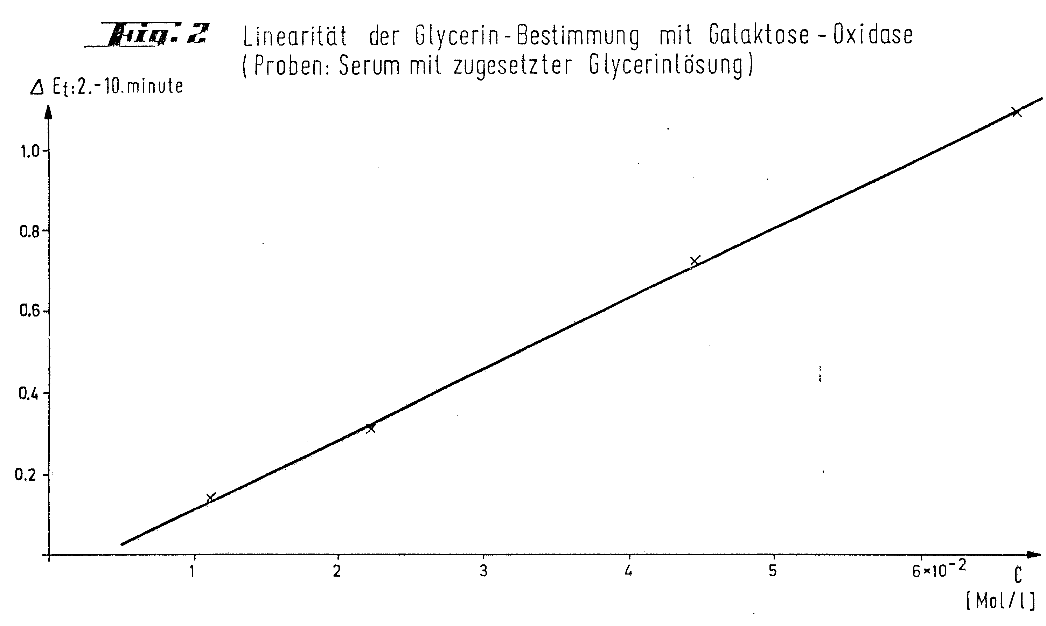

2,0 ml Reagens in Küvette pipettieren, 0,1 ml Probe zusetzen und mischen. Nach Beendigung der lag-Phase wird die Extinktionsänderung von der zweiten bis zur zehnten Minute registriert. Die lineare Abhängigkeit zwischen Glycerinkonzentration und Extinktionsänderung zeigt Fig. 2 der Zeichnung.

## Beispiel 3

| Reagens: | | |
|---|---|---|
| Glycylglyin-Puffer, pH 8,0; | 0,1 Mol/l | |
| $(NH_4)_2SO_4$ | 0,16 Mol/l | |
| $ZnSO_4 \cdot 7\,H_2O$ | 0,1 mMol/l | |
| $Na_2CO_3$ | 0,078 Mol/l | |
| 4-Aminoantipyrin-$NH_2$ | 0,5 mMol/l | |

| Reagens: | | |
|---|---|---|
| p-Chlorphenol | 10 mMol/l | |
| POD | 10 000 U/l | |
| LDH | 10 000 U/l | |
| Pyruvat | 2,25 mMol/l | |
| Gal-OD | 20 000 U/l | |
| Glyc-DH | 50 000 U/l | |
| $NAD^+$ | 1,5 mMol/l | |

## Probe

Wäßrige Glycerinlösungen im Bereich von 0,001 bis 0,01 Mol/l (statt der wäßrigen Glycerinlösungen können Serum, andere Körperflüssigkeiten und auch beispielsweise Lebensmittelextrakte eingesetzt werden).

## Bestimmungsansatz

| Meßstrahlung | 546 nm |
|---|---|
| Schichtdicke | 1 cm |
| Temperatur | 25° C bis 37° C |

1,0 ml Reagens in die Küvette geben und mit 0,01 ml Probe starten. Extinktion nach 30 Minuten gegen Reagensleerwert messen. Für die quantitative Bestimmung wird dieses $\Delta$ E über einen Standard zur Glycerinkonzentration in Beziehung gesetzt.

Die Mitführung eines Probenleerwerts ist nicht erforderlich. Eine durch Auftragen der Glycerinkonzentration gegen die Extinktion bei 546 nm erhaltene Kurve ist in Abbildung 3 wiedergegeben.

## Patentansprüche

1. Verfahren zur Bestimmung von Glycerin, dadurch gekennzeichnet, daß Glycerin in wäßrigem Medium in Gegenwart von Sauerstoff mit Galactoseoxidase inkubiert und entweder der Sauerstoffverbrauch oder gebildetes $H_2O_2$ bzw. Glycerinaldehyd bestimmt werden.

2. Verfahren nach Anspruch 1, dadurch gekennzeichnet, daß der Sauerstoffverbrauch polarographisch gemessen wird.

5

3. Verfahren nach Anspruch 1, dadurch gekennzeichnet, daß gebildetes H₂O₂ enzymatisch mit Katalase oder Peroxidase bestimmt wird.

4. Verfahren nach Anspruch 3, dadurch gekennzeichnet, daß man Peroxidase und ein Chromogen zusetzt und die Färbung bzw. Extinktionsänderung bestimmt.

5. Verfahren nach einem der Ansprüche 1 bis 4, dadurch gekennzeichnet, daß man die Messung kinetisch in einem vorbestimmten kurzen Zeitintervall durchführt.

6. Verfahren nach einem der Ansprüche 1 bis 5, dadurch gekennzeichnet, daß man zur Inkubation auch Glycerindehydrogenase und NAD zusetzt.

7. Verfahren nach Anspruch 6, dadurch gekennzeichnet, daß man NAD zusammen mit einem NAD-regenerierenden System zusetzt.

8. Verfahren nach Anspruch 7, dadurch gekennzeichnet, daß man als NAD-regenerierendes System Pyruvat und Lactatdehydrogenase verwendet.

9. Verfahren nach einem der vorhergehenden Ansprüche, dadurch gekennzeichnet, daß man bei pH 7,0 bis 9,0 arbeitet.

10. Verfahren nach Anspruch 1, dadurch gekennzeichnet, daß gebildeter Glycerinaldehyd mit einem Hydrazinderivat zum entsprechenden Hydrazon umgesetzt und letzteres gemessen wird.

11. Reagens zur Bestimmung von Glycerin, dadurch gekennzeichnet, daß es aus Galactoseoxidase und einem System zur Bestimmung von H₂O₂ oder einem System zur Bestimmung von Glycerinaldehyd besteht.

12. Reagens nach Anspruch 11, dadurch gekennzeichnet, daß zusätzlich ein Mittel zur Verseifung von verestertem Glycerin enthält.

13. Reagens nach Anspruch 11 oder 12, dadurch gekennzeichnet, daß das System zur Bestimmung von H₂O₂ aus Peroxidase, wenigstens einem Chromogen und Puffer besteht.

14. Reagens nach Anspruch 13, dadurch gekennzeichnet, daß es 0,5 bis 100 U/ml Peroxidase, 5 bis 35 U/ml Galactoseoxidase, 0,05 bis 0,2 mMol/ml Puffer, pH 6 bis 9, und 0,05 bis 20 µMol/ml Chromogen enthält.

15. Reagens nach einem der Ansprüche 11 bis 14, dadurch gekennzeichnet, daß es zusätzlich Glycerindehydrogenase, NAD und ein NAD-regenerierendes System enthält.

16. Reagens nach Anspruch 15, dadurch gekennzeichnet, daß das NAD-regenerierende System aus Pyruvat und Lactatdehydrogenase besteht.

17. Reagens nach Anspruch 14 und 15, dadurch gekennzeichnet, daß es 5 bis 100 U/ml Glycerindehydrogenase und wenigstens 0,05 µMol/ml NAD enthält.

18. Reagens nach einem der Ansprüche 11 bis 17, dadurch gekennzeichnet, daß das Chromogen aus p-Chlorphenol und 4-Aminoantipyrinamid besteht.

19. Reagens nach Anspruch 18, dadurch gekennzeichnet, daß es 5 bis 15 µMol/ml p-Chlorphenol und 0,1 bis 1,0 µMol/ml 4-Aminoantipyrinamid enthält.

20. Reagens nach einem der Ansprüche 13 bis 19, dadurch gekennzeichnet, daß das Chromogen aus 2,2'-Azinodi-(3-äthyl-benzthiazolinsulfonsäure-6)-diammoniumsalz besteht.

21. Reagens nach einem der Ansprüche 11 bis 20, dadurch gekennzeichnet, daß es Glycylglycinpuffer oder Bicinpuffer, pH 7,0 bis 9,0, enthält.

22. Reagens nach einem der Ansprüche 11 bis 21, dadurch gekennzeichnet, daß es zusätzlich ein oberflächenaktives Mittel enthält.

23. Reagens nach einem der Ansprüche 11 bis 22, dadurch gekennzeichnet, daß es auf einem Trägermaterial, wie Papier, imprägniert vorliegt.

24. Verfahren nach Anspruch 6, dadurch gekennzeichnet, daß reduziertes NAD bestimmt wird.

## Claims

1. Process for the determination of glycerol, characterised in that glycerol is incubated in an aqueous medium in the presence of oxygen with galactose oxidase and either the oxygen consumption or the H₂O₂ or glyceraldehyde formed are determined.

2. Process according to claim 1, characterised in that the oxygen consumption is measured polarigraphically.

3. Process according to claim 1, characterised in that the H₂O₂ formed is determined enzymatically with catalase or peroxidase.

4. Process according to claim 3, characterised in that one adds peroxidase and a chromogen and determines the coloration or change of extinction.

5. Process according to one of preceding claims 1 to 4, characterised in that one carries out the measurement kinetically in a predetermined short time interval.

6. Process according to one of claims 1 to 5, characterised in that, for the incubation, one also adds glycerol dehydrogenase and NAD.

7. Process according to claim 6, characterised in that one adds NAD, together with an NAD-regenerating system.

8. Process according to claim 7, characterised in that one uses pyruvate and lactate dehydrogenase

# 0 048 347

as NAD-regenerating system.

9. Process according to one of the preceding claims, characterised in that one works at pH 7.0 to 9.0.

10. Process according to claim 1, characterised in that glyceraldehyde formed is reacted with a hydrazine derivative to give the corresponding hydrazone and the latter is measured.

11. Reagent for the determination of glycerol, characterised in that it consists of galactose oxidase and a system for the determination of $H_2O_2$ or a system for the determination of glyceraldehyde.

12. Reagent according to claim 11, characterised in that it additionally contains an agent for the saponification of esterified glycerol.

13. Reagent according to claim 11 or 12, characterised in that the system for the determination of $H_2O_2$ consists of peroxidase, at least one chromogen and buffer.

14. Reagent according to claim 13, characterised in that it contains 0.5 to 100 U/ml. peroxidase, 1 to 35 U/ml. galactose oxidase, 0.05 to 0.2 mMol/ml. buffer, pH 6 to 9, and 0.05 to 20 µMol/ml. of chromogen.

15. Reagent according to one of claims 11 to 14, characterised in that it additionally contains glycerol dehydrogenase, NAD and a NAD-regenerating system.

16. Reagent according to claim 15, characterised in that the NAD-regenerating system consists of pyruvate and lactate dehydrogenase.

17. Reagent according to claims 14 and 15, characterised in that it contains 5 to 100 U/ml. glycerol dehydrogenase and at least 0.05 µMol/ml. NAD.

18. Reagent according to one of claims 11 to 17, characterised in that the chromogen consists of p-chlorophenol and 4-aminoantipyrinamide.

19. Reagent according to claim 18, characterised in that it contains 5 to 15 Mol/ml. p-chlorophenol and 0.1 to 1.0 µMol/ml. 4-aminoantipyrinamide.

20. Reagent according to one of claims 13 to 19, characterised in that the chromogen consists of 2,2'-azinodi-(3-ethylbenzthiazoline-6-sulphonic acid) diammonium salt.

21. Reagent according to one of claims 11 to 20, characterised in that it contains glycylglycine buffer or bicine buffer, pH 7.0 to 9.0.

22. Reagent according to one of claims 11 to 21, characterised in that it additionally contains a surface-active agent.

23. Reagent according to one of claims 11 to 22, characterised in that it is present impregnated on to a carrier material, such as paper.

24. Process according to claim 6, characterised in that reduced NAD is determined.


**Revendications**

1. Procédé de dosage du glycérol, caractérisé en ce qu'on fait incuber du glycérol en milieu aqueux en présence d'oxygène avec de la galactoseoxydase et en ce qu'on détermine soit la consommation d'oxygène, soit le $H_2O_2$ ou le glycéraldéhyde formé.

2. Procédé suivant la revendication 1, caractérisé en ce qu'on mesure la consommation d'oxygène polarographiquement.

3. Procédé suivant la revendication 1, caractérisé en ce qu'on dose enzymatiquement le $H_2O_2$ formé avec de la catalase ou de la peroxydase.

4. Procédé suivant la revendication 3, caractérisé en ce qu'on ajoute de la peroxydase et un chromogène, et en ce qu'on détermine la variation de coloration ou respectivement d'extinction.

5. Procédé suivant l'une des revendications 1 à 4, caractérisé en ce qu'on effectue la mesure cinétiquement dans un intervalle de temps court, déterminé à l'avance.

6. Procédé suivant l'une des revendications 1 à 5, caractérisé en ce que, pour l'incubation, on ajoute également de la glycéroldeshydrogénase et du NAD.

7. Procédé suivant la revendication 6, caractérisé en ce qu'on ajoute du NAD en même temps qu'un système régénérant le NAD.

8. Procédé suivant la revendication 7, caractérisé en ce qu'on utilise comme système régénérant le NAD du pyruvate et de la lactate deshydrogénase.

9. Procédé suivant l'une des revendications précédentes, caractérisé en ce qu'on opère à un pH de 7,0 à 9,0.

10. Procédé suivant la revendication 1, caractérisé en ce qu'on fait réagir le glycéraldéhyde formé avec un dérivé de l'hydrazine pour donner l'hydrazone correspondante et en ce qu'on mesure cette dernière.

11. Réactif pour le dosage du glycérol, caractérisé en ce qu'il se compose de galactoseoxydase et d'un système pour le dosage de $H_2O_2$ ou d'un système pour le dosage du glycéraldéhyde.

12. Réactif suivant la revendication 11, caractérisé en ce qu'il contient en outre un agent pour la saponification du glycérol estérifié.

13. Réactif suivant la revendication 11 ou 12, caractérisé en ce que le système pour le dosage de $H_2O_2$ se compose de peroxydase, d'au moins un chromogène et de tampon.

14. Réactif suivant la revendication 13, caractérisé en ce qu'il contient 0,5 à 100 U/ml de peroxydase,

7

5 à 35 U/ml de galactoseoxydase, 0,05 à 0,2 mmole/ml de tampon, pH 6 à 9, et 0,05 à 20 µmole/ml de chromogène.

15. Réactif suivant l'une des revendications 11 à 14, caractérisé en ce qu'il contient en outre de la glycéroldeshydrogénase, du NAD et un système régénérant le NAD.

16. Réactif suivant la revendication 15, caractérisé en ce que le système régénérant le NAD se compose de pyruvate et de lactate deshydrogénase.

17. Réactif suivant les revendications 14 et 15, caractérisé en ce qu'il contient 5 à 100 U/ml de glycéroldeshydrogénase et au moins 0,05 µmole/ml de NAD.

18. Réactif suivant l'une des revendications 11 à 17, caractérisé en ce que le chromogène est constitué de p-chlorophénol et de 4-aminoantipyrinamide.

19. Réactif suivant la revendication 18, caractérisé en ce qu'il contient 5 à 15 µmole/ml de p-chlorophénol et 0,1 à 1,0 µmole/ml de 4-aminoantipyrinamide.

20. Réactif suivant l'une des revendications 13 à 19, caractérisé en ce que le chromogène se compose de sel diammonique de l'acide 2,2'-azinodi-(3-éthyl-benzothiazoline 6-sulfonique).

21. Réactif suivant l'une des revendications 11 à 20, caractérisé en ce qu'il contient du tampon glycylglycine ou du tampon bicine, pH 7,0 à 9,0.

22. Réactif suivant l'une des revendications 11 à 21, caractérisé en ce qu'il contient en outre un agent tensioactif.

23. Réactif suivant l'une des revendications 11 à 22, caractérisé en ce qu'il est présent en imprégnation sur une matière support telle que le papier.

24. Procédé suivant la revendication 6, caractérisé en ce qu'on dose le NAD réduit.

**Fig. 1** Linearität der Glyzerin-Bestimmung mit Galaktose-Oxidase (Proben: wäßrige Glyzerinlösungen)

**Fig. 2** Linearität der Glycerin-Bestimmung mit Galaktose-Oxidase
(Proben: Serum mit zugesetzter Glycerinlösung)

Fig. 3